Europäisches Patentamt

European Patent Office

Office européen des brevets

(19)

(11) **EP 1 082 091 B1**

(12) **FASCICULE DE BREVET EUROPEEN**

(45) Date de publication et mention
de la délivrance du brevet:
**27.03.2002   Bulletin 2002/13**

(21) Numéro de dépôt: **99922249.0**

(22) Date de dépôt: **02.06.1999**

(51) Int Cl.⁷: **A61K 7/32**, A61K 7/48

(86) Numéro de dépôt international:
**PCT/FR99/01286**

(87) Numéro de publication internationale:
**WO 99/62473 (09.12.1999 Gazette 1999/49)**

(54) **UTILISATION D'EXTRAITS DE MICROORGANISMES DANS DES COMPOSITIONS COSMETIQUES OU PHARMACEUTIQUES**

VERWENDUNG VON MIKROORGANISMEN EXTRAKTEN IN KOSMETISCHEN ODER PHARMAZEUTISCHEN ZUSAMMENSETZUNGEN

USE OF MICRO-ORGANISM EXTRACTS IN COSMETIC OR PHARMACEUTICAL COMPOSITIONS

(84) Etats contractants désignés:
**AT BE CH CY DE DK ES FI FR GB GR IE IT LI LU MC NL PT SE**

(30) Priorité:  **03.06.1998  FR 9806957**

(43) Date de publication de la demande:
**14.03.2001   Bulletin 2001/11**

(73) Titulaire: **Pierre Fabre Dermo-Cosmetique 92100 Boulogne-Billancourt (FR)**

(72) Inventeur: **LAGARDE, Isabelle F-31540 Maurens (FR)**

(74) Mandataire: **Ahner, Francis et al Cabinet Régimbeau 20, rue de Chazelles 75847 Paris cedex 17 (FR)**

(56) Documents cités:
**EP-A- 0 688 562          DE-C- 4 231 544**
**FR-A- 2 051 697          FR-A- 2 352 057**
**FR-A- 2 700 470          LU-A- 41 888**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présenté invention a pour objet l'utilisation d'extraits de microorganismes, en particulier les bactéries Gram+, pour la préparation de compositions cosmétiques ou pharmaceutiques destinées à inhiber les odeurs corporelles. Elle a également pour objet lesdites compositions cosmétiques ou pharmaceutiques.

L'invention concerne la lutte contre odeurs corporelles et en particulier celles d'origine axillaire, périnéale et des pieds.

Afin de prévenir la production d'odeurs corporelles d'origine microbienne, et notamment des microorganismes Gram+, quatre types de composés sont actuellement utilisés :

- des molécules à propriété antimicrobienne vis-à-vis des Gram+, éventuellement dans un excipient hydroalcoolique,
- des molécules astringeantes vis-à-vis des pores cutanés, qui ont pour effet de lutter contre une transpiration excessive,
- des esters d'acide hydroxy-carboxylique qui abaissent le pH cutané et favorisent l'inhibition des estérases bactériennes responsables de l'hydrolyse des lipides contenus dans la sueur apocrine, ou
- des adsorbants chimiques qui captent les composés volatils, tels que les mercaptans et les amines, issus de la dégradation physiologique du stratum corneum par la flore cutanée.

Cependant, les compositions de l'art antérieur comprenant ces composés présentent notamment les inconvénients de perturber la flore locale, de favoriser l'émergence de mycoses cutanées et/ou de diminuer la sudation naturelle dûe au rôle excréteur de la peau. De plus, les formulations acides sont limitantes et peuvent aussi favoriser l'émergence de mycoses et les capteurs d'odeurs captent aussi le parfum de la formule.

La présente invention se propose donc de remédier aux inconvénients de l'art antérieur et de prévenir efficacement les odeurs corporelles, notamment d'origine axillaire, périnéale et des pieds par l'utilisation d'extraits de microorganismes pour la préparation de compositions cosmétiques ou pharmaceutiques destinées à inhiber lesdites odeurs.

(FR 2 700 470 a pour objet des compositions cosmétiques contenant des bactéries inactivées et/ou des parois bactériennes ainsi que leur utilisation, afin d'améliorer le système de défense de la peau. Il n'y est nullement question d'inhibition des odeurs corporelles.

FR 2 051 697 concerne des compositions cosmétiques comprenant des protéines issues de microorganismes obtenus par fermentation dans un milieu nutritif comprenant, comme source de carbone, un hydrocarbure de pétrole. Lesdites compositions cosmétiques contiennent donc comme ingrédient une protéine, ce qui diffère des composés de surface et/ou exocellulaires tels qu'utilisés dans le cadre de la présente invention.)

La flore axillaire normale est essentiellement composée de bacilles et coques Gram+.

Chez 50 jeunes adultes sains, la prévalence de corynébactéries lipophiles (Mac Guiley, 1985) est de 66 % avec une densité axillaire de $4,66 \pm 1,48$ (Log $_{10}$) par cm². Au niveau des espaces interdigitaux des pieds, la prévalence atteint 98 % avec une densité de $6,22 \pm 1,50$. Elle est de 66 % au niveau des aisselles pour une densité de $4,66 \pm 1,48$.

Une étude de Noble (1981), montre que la prévalence de *Corynebacterium xerosis* en fonction de l'humidité corporelle, chez 30 jeunes adultes, est de 47 % avec une densité de 6,66 Log 10 au niveau périnéal (par cm²), de 20 % au niveau des espaces interdigitaux des orteils pour une densité de 6,53 Log $_{10}$ et de 7 % au niveau des aisselles avec une densité de 3,19 Log $_{10}$.

La corrélation quantitative entre la forte odeur axillaire et la présence de bactéries corynéformes a été réalisée (Leyden et al.). Plus particulièrement Rennie (1991) a démontré in vitro que l'odeur axillaire était dûe à la présence de *Corynebacterium xerosis.*

Par ailleurs, les germes cutanés ont une capacité d'adhésion aux cellules épithéliales humaines et cette capacité a pu être mise en évidence par Romero-Steiner (1990).

Les extraits de microorganismes, en particulier les Gram+, utilisés dans le cadre de la présente invention agissent par inhibition compétitive de l'attachement bactérien au récepteur cellulaire de l'épiderme de façon à le saturer artificiellement. En effet, dans des conditions normales, les bactéries qui se trouvent dans un environnement propice à son développement, notamment en terme de température et d'humidité, vont se fixer sur des récepteurs de l'épiderme. Cette fixation va permettre aux bactéries de se multiplier et ainsi de donner lieu au développement d'odeurs corporelles.

La présente invention propose d'amener *in situ*, préalablement à la fixation des bactéries sur les récepteurs, des composés de surface et/ou exocellulaires de microorganismes, notamment Gram+, qui vont se comporter comme des analogues structuraux du site d'attachement de la bactérie au récepteur cellulaire, qui vont se fixer sur ce récepteur et donc le rendre inaccessible aux bactéries susceptibles de se développer à ce niveau.

Il ne s'agit donc pas là d'utiliser à proprement parler un anti-microbien au sens classique du terme (par exemple un antiseptique ou un antibiotique) mais un composé d'origine microbienne qui agit au niveau environnemental du germe.

En effet, la Demanderesse a mis en évidence de façon surprenante l'effet de l'inhibition compétitive de l'adhésion bactérienne par les susdits analogues structuraux du site d'attachement de la bactérie.

Par "composés de surface", on entend les structures biochimiques (lipides, glucides, protéines) entrant dans la composition de la surface des bactéries.

Par "composés exocellulaires", on entend les structures biochimiques (lipides, glucides, protéines) entrant dans la composition de la surface des bactéries et / ou excrétée par celles-ci dans le milieu ambiant compatible avec leur survie.

Par "inhibition des odeurs corporelles", on entend une diminution voire une totale suppression des odeurs corporelles qui se développeraient chez un sujet normal ne subissant aucun traitement destiné à combattre localement lesdites odeurs.

La cinétique d'attachement de germes cutanés aux surfaces cellulaires est en général très rapide et *Corynebacterium xerosis* (ATCC 5216) par exemple adhère en 4 heures à la surface de 15-20 % de kératinocytes HACAT (15 à 20 % d'adhésion).

Par contre, *Corynebacterium xerosis* ayant subi un traitement physique lui otant les composés de surface, n'adhère pas aux kératinocytes HACAT.

De même, divers antiseptiques ou conservateurs ou molécules à propriétés antimicrobienne vis-à-vis des Gram+ incubés à une concentration sub inhibitrice ($\frac{CMI}{3}$) favorisent une diminution de l'attachement microbien (Irgasan® 0,0001 %, Rétinal 0,0001 %, CTAB 0,0001 %, digluconate de Chlorhexidine 0,0001 %).

Ainsi, l'invention a également pour objet l'utilisation d'extraits de microorganismes Gram+ tels que définis plus haut, les microorganismes étant en particulier des corynébactéries choisies de préférence dans le groupe comprenant : *Corynebacterium xerosi, Corynebacterium minutissum*, les corynébactéries lipophiles des groupes CLC, JK et D2 et autres bactéries corynéformes retrouvées sur la peau, telles que *Dermabacter hominis* et *Brevibacterium species*.

La présente invention a également pour objet l'utilisation d'extraits de corynébactéries pour la préparation de compositions cosmétiques ou pharmaceutiques, les extraits en question comprenant comme ci-dessus indiqué des composés de surface et exocellulaires des corynébactéries, elles-mêmes choisies dans le groupe ci-dessus défini.

Enfin, la présente invention concerne des compositions cosmétiques ou pharmaceutiques comprenant des extrait de corynébactéries et en particulier des composés de surface et exocellulaires de corynébactéries. Ces dernières sont choisies dans le groupe ci-dessus défini. Les compositions cosmétiques ou pharmaceutiques conformes à l'invention comprennent lesdits extraits de corynébactéries en une quantité comprise entre 0,01 % et 20 % et de préférence entre 0,5 et 5 % en poids par rapport à la composition totale.

Les compositions conformes à l'invention peuvent se présenter sous la forme de déodorants, spray déodorant, crème déodorante.....

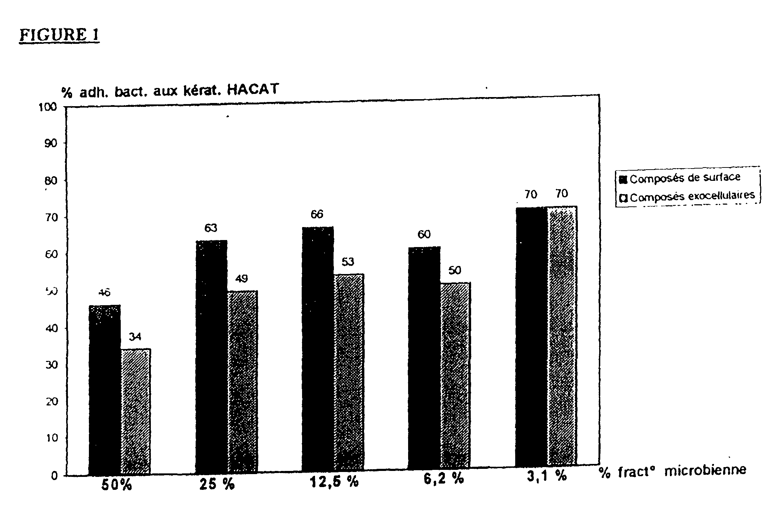

La figure 1 présente le pourcentage d'adhésion de *Corynebacterium xerosis* (ATCC 5216) aux kératinocytes HACAT après incubation des fractions microbiennes des bactéries et des cellules pendant deux heures (fractions microbiennes issues d'une biomasse réalisée dans un milieu riche). Les composés de surface ont été obtenus par agitation mécanique..

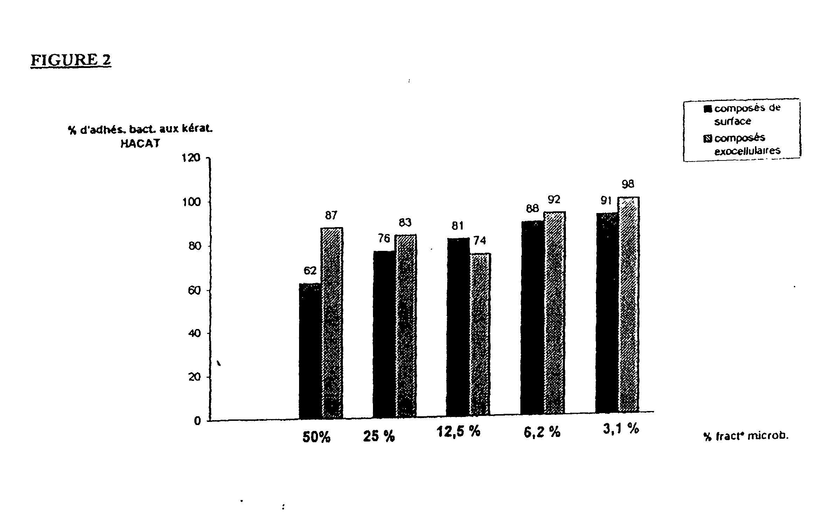

La figure 2 présente le pourcentage d'adhésion de *Corynebacterium xerosis* (ATCC 5216) aux kératinocytes HACAT après incubation des fractions microbiennes des bactéries et des cellules pendant deux heures (fractions microbiennes issues d'une biomasse réalisée dans un milieu saccharosé). Les composés de surface ont été obtenus par agitation mécanique.

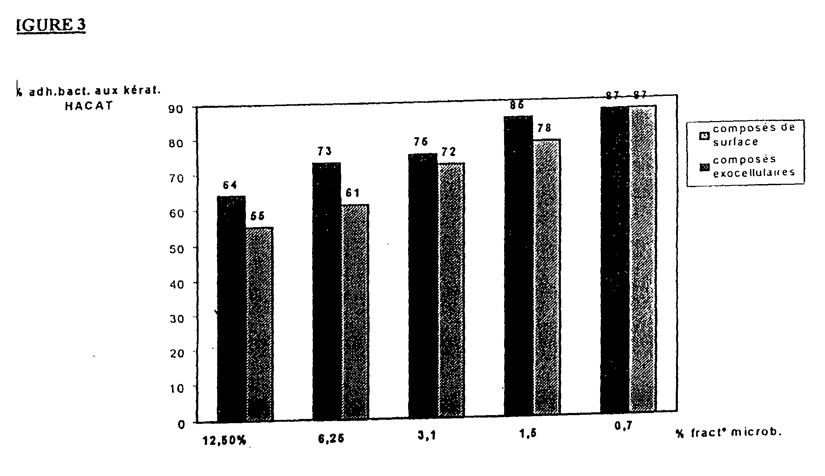

La figure 3 présente le pourcentage d'adhésion de *Corynebacterium xerosis* (ATCC 5216) aux kératinocytes HACAT après incubation des fractions microbiennes des bactéries et des cellules pendant quatre heures. Les composés de surface ont été obtenus par agitation mécanique.

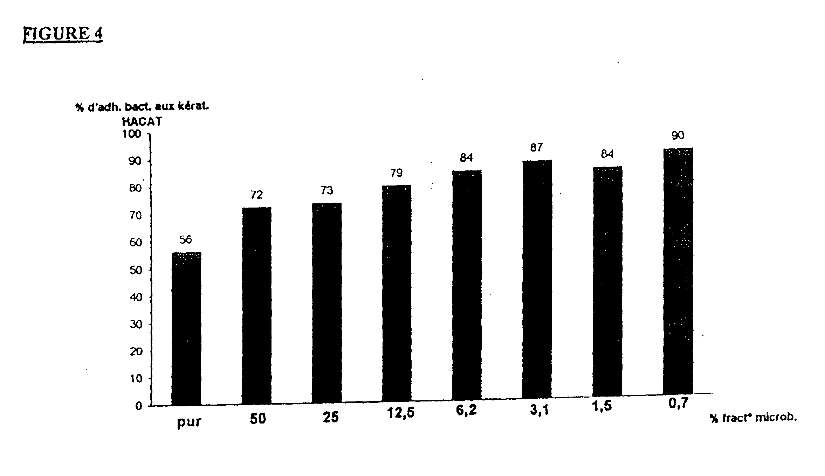

La figure 4 présente le pourcentage d'adhésion de *Corynebacterium xerosis* (ATCC 5216) aux kératinocytes HACAT après incubation des composés de surface de *C.xerosis* (ATCC 5216) et des cellules pendant quatre heures (fractions microbiennes obtenues après traitement de la biomasse de *C.xerosis* (ATCC 5216) par la chaleur).

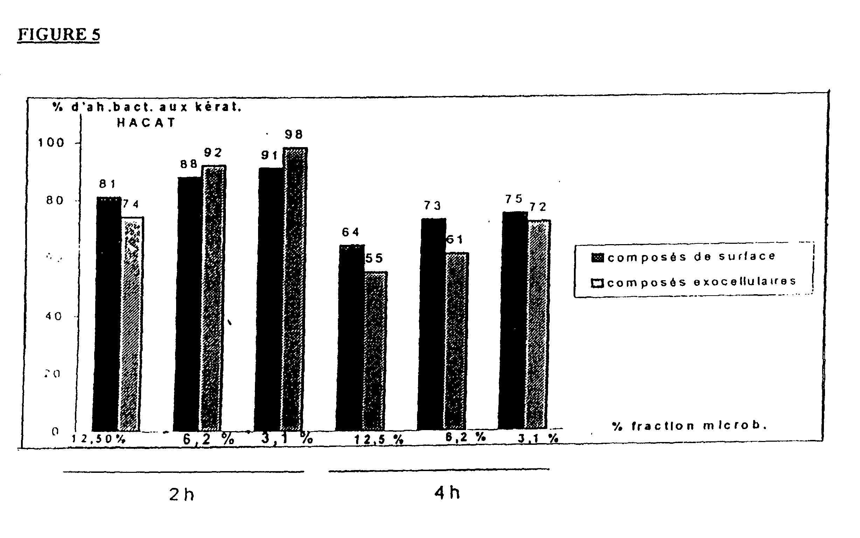

La figure 5 illustre l'inhibition de l'adhésion de *C.xerosis* (ATCC 5216) par les deux types de composés microbiens obtenus de façon mécanique avec efficacité en fonction du temps. Cette inhibition est présentée sur une période de 2 ou 4 heures pour des concentrations en composés microbiens de 3, 1 à 12,5 %.

Le phénomène d'inhibition de l'adhésion bactérienne a été vérifié par marquage de *Corynebacterium xerosis* (ATCC 5216) avec 20 µCi de thymidine tritiée et incubation à 37°C pendant 18 heures de façon à obtenir un inoculum marqué.

Après lavage de l'inoculum bactérien et préparation d'un tapis cellulaire défini de kératinocytes HACAT (kératinocytes humains mutés spontanément, voir Bonkamp et al., 1988), $10^8$ bactéries par ml sont ajoutées dans chaque puits de plaque cellulaire contenant le tapis de kératinocytes.

Différentes fractions bactériennes ont été testées. Les meilleurs résultats ont été obtenus à l'aide de composés exocellulaires et des composés de surface à partir de culture de *Corynebacterium xerosis* dans un bouillon Trypticase soja. Les extraits bactériens inhibiteurs ont été obtenus par léger autoclavage de la suspension bactérienne débarrassée de Bouillon Trypticase soja (centrifugation et lavage). Un autoclavage de 5 à 15 minutes à 100°C permet d'obtenir les fractions du composé de surface pratiquement intactes et avec un bon rendement. Sa mise en suspension dans un excipient type propylène ou butylène glycol, assure une bonne protection microbienne sans ajout complémentaire de conservateur.

L'essai d'inhibition de l'adhésion proprement dit, a été réalisé de la dilution ½ (limite maximale du test) jusqu'à la dilution $\frac{1}{32}$ pendant 2 heures à 37°C. Les résultats montrent que les fractions obtenues à partir d'un milieu riche (type Trypticasc soja) plutôt qu'un milieu pauvre (saccharose avec quelques ions nutritifs) inhibent significativement l'adhésion des bactéries aux kératinocytes (figures 1 et 2). Les composés exocellulaires excrétés par *Corynebacterium xerosis* sont très proches biochimiquement des composés de la surface de la bactérie (analyses des acides gras par chromatographie sur couche mince et gazeuse, analyse des glycolipides, électrophorése des protéines de surface et exocellulaires et profil chromatographique des fractions polysaccharadiques des composés de surface ct exocellulaires).

L'inhibition de l'adhésion a lieu aussi fortement dans le cas des fractions exocellulaires que des fractions situées à la surface de *Corynebacterium xerosis*, que la séparation entre les composés de surface et le corps de la bactérie soit réalisé par agitation mécanique ou bien par autoclavage (figures 3 et 4).

L'avantage de ce type de traitement par rapport à celui proposé par le brevet de Sagami Central Chemical Research Institute (6-135843 (1994)) est qu'on obtient des fractions bactériennes correspondant aux analogues struturaux du site d'attachement de la bactérie au récepteur car il s'agit de la paroi bactérienne, qu'elle soit constitutive ou bien relarguée dans le milieu, et non d'un lysat enzymatique microbien qu'il convient de purifier. De plus, une lyse microbienne comme proposé dans le brevet Sagami, produit de nombreuses substances biochimiques susceptibles de réagir entre elles et de ne pas inhiber l'adhésion de façon optimale.

Par ailleurs, la technique ci-dessus décrite de mise en évidence à l'aide d'incorporation d'un élément radioactif est fine, reproductible et statistiquement interprétable par le test de Student. Les essais préliminaires réalisés avec la technique de Coie et Silverberg (1986) dont une variante est présente dans le brevet japonais, a montré cette limite.

Les extraits de microorganismes, et en particulier de bactéries Gram+, préparés comme décrits ci-dessus peuvent être utilisés comme principes actifs dans des formules diverses et les exemples suivants ne sont donnés qu'à titre illustratif.

## EXEMPLES

| Exemple 1 : déodorant | |
| --- | --- |
| Composés de surface et exocellulaires de corynébactérie cutanée | 0,01 - 20 % |
| Alcool dénaturé | 20 - 60 % |
| Propylène glycol | 1 % - 20 % |
| Diméthicone copolyol | 1 % - 5 % |
| Stéarate de soude | 3 % - 10 % |
| Parfum | 0,1 - 1% |
| Eau | QSP |

| Exemple 2 : déodorant | |
| --- | --- |
| Composés de surface et exocellulaires de corynébactérie cutanée | 0,01 - 20 % |
| Propylène glucol | 10 % - 60 % |
| Stéarate de soude | 3 % - 10 % |
| Hydroxyéthylcellulose | 0,1 % - 3 % |
| Chlorure d'isostéaramidopropyl PG dimonium | 1 % - 3 % |
| Lauramide DEA | 0,5 % - 5 % |
| Parfum | 0,1 - 1 % |
| Eau | QSP |

| Exemple 3 : déodorant sous forme de spray | |
|---|---|
| Composés surface et exocellulaires de corynébactérie cutanée | 0,01 - 20 % |
| Alcool | 5 - 40 % |
| PEG 7 - Glycéril-cocoate | 0,5 - 5 % |
| Chlorhydrate aluminium | 1 - 40 % |
| Parfum | 0,1 - 1 % |
| Diméticone copolyol | 1 - 5 % |
| Eau | QSP |

| Exemple 4 : crème déodorante | |
|---|---|
| Composés de surface et exocellulaires spécifiques de Corynébactérie cutanée | 0,01 - 20 % |
| Cétéareth®-12 | 1 - 5 % |
| Cétéareth®-20 | 1 - 5 % |
| Alcool cetearylique | 0,5 - 3 % |
| Octyldodécanol | 1 - 5 % |
| Eau | QSP |

**REFERENCES**

- Bonkamp P., Petrussevska R.T., Breitkreutz D., Hourung J., Markham A. and Fusenig N.E. Normal keratinization in spontaneously immortalized aneuploid human keratinized cell line. The Journal of Cell Biology, vol. 106, Mars 1988, 761 - 771.

- Cole G.W., Silverberg N.L. The adherence of Staphylococcus aureus to human corneocyts Arch. Dermatol. (19886), 122, 166 - 169.

- Leyden J.J., Mc Gingley K.J., Hoelzle E., Labows J.N., Kligman A.M. The microbiology of the human axilla and its relationship to axillary odor. J. Invest. Dermatol. 1981 ; 77 : 413 - 16

- Mac Ginley et col. Analysis of cellular components, biochemical reactions and habitat of human cutancous lipophilic diphteroids. J. Invest Dermatol 1985 85, p. 374-377

- Noble W.C., Microbiology of human skin 2nd ed London Lloyd-Lube 1981.

- Rennie P.J., Gower D.B., Holland K.T. In vitro and in vivo studies of human axillary odour and the cutaneous microflora. Br. J. Dermatol. 1991 Jun, vol. 124 (6), p 596 - 602.

- Romero - Steiner S., Witek. T, Balish E. Adherence of skin bacteria to human epithelial cells, J. of Clin. Microb. Jan. 1990 : 27 - 31

**Revendications**

1. Utilisation d'extraits de microorganismes constitués de composés de surface et/ou exocellulaires desdits microorganismes pour la préparation de compositions cosmétiques ou pharmaceutiques destinées à inhiber les odeurs corporelles que sont les odeurs axillaires, périnéales et des pieds, par saturation des sites d'attachement des bactéries aux récepteurs de l'épiderme.

2. Utilisation d'extraits de microorganismes selon la revendication 1, **caractérisée en ce qu'**il s'agit d'extraits de microorganismes Gram+,

**3.** Utilisation d'extraits de microorganismes selon la revendication 1 ou 2, **caractérisée en ce que** les microorganismes sont des corynébactéries ou autres bactéries corynéformes retrouvées sur la peau.

**4.** Utilisation d'extraits de microorganismes selon la revendication 3, **caractérisée en ce que** les corynébactéries sont choisies dans le groupe comprenant *Corynebacterium xerosis, Corynebacterium minutissum* et les corynébactéries lipophiles des groupes CLC, JK et D2.

**5.** Utilisation d'extraits de microorganismes selon la revendication 3, **caractérisée en ce que** les bactéries corynéformes retrouvées sur la peau sont choisies dans le groupe comprenant *Dermabacter hominis* et *Brevibacterium species*.

**6.** Compositions cosmétiques ou pharmaceutiques comprenant des extraits de corynébactéries ou autres bactéries corynéformes retrouvées sur la peau tels que définis dans la revendication 4 ou 5, **caractérisées en ce qu'**elles sont destinées à l'inhibition des odeurs corporelles.

**7.** Compositions cosmétiques ou pharmaceutiques selon la revendication 6, **caractérisées en ce qu'**elles comprennent des composés de surface et/ou exocellulaires de corynébactéries.

**8.** Compositions cosmétiques ou pharmaceutiques selon l'une des revendications 6 et 7, **caractérisées en ce que** les corynébactéries sont choisies dans le groupe comprenant : *Corynebacterium xerosis, Corynebacterium minutissum* et les corynébactéries lipophiles des groupes CLC, JK et D2.

**9.** Compositions cosmétiques ou pharmaceutiques selon la revendication 6, **caractérisées en ce que** les bactéries corynéformes retrouvées sur la peau sont choisies dans le groupe comprenant *Dermabacter hominis* et *Brevibacterium species*.

**10.** Compositions cosmétiques ou pharmaceutiques selon l'une des revendications 6 à 8, **caractérisées en ce qu'**elles comprennent des extraits de corynébactéries en une quantité comprise entre 0,01 % et 20 % en poids par rapport à la composition totale.

**11.** Compositions cosmétiques ou pharmaceutiques selon la revendication 10, **caractérisées en ce qu'**elles comprennent des extraits de corynébactéries en une quantité comprise entre 0,5 et 5 % en poids par rapport à la composition totale.

## Patentansprüche

**1.** Verwendung von Extrakten von Mikroorganismen, die zusammengesetzt sind aus Oberflächen- und/oder exozellulären Verbindungen der Mikroorganismen, für die Herstellung von kosmetischen oder pharmazeutischen Zusammensetzungen, die dazu bestimmt sind, Körpergerüche, bei denen es sich um die Achsel-, perinealen und Fußgerüche handelt, durch Sättigung der Stellen der Anhaftung von Bakterien an den Rezeptoren der Epidermis zu hemmen.

**2.** Verwendung von Extrakten von Mikroorganismen nach Anspruch 1, **dadurch gekennzeichnet, dass** es sich um Extrakte von grampositiven Mikroorganismen handelt.

**3.** Verwendung von Extrakten von Mikroorganismen nach Anspruch 1 oder 2, **dadurch gekennzeichnet, dass** die Mikroorganismen Corynebakterien oder andere coryneforme Bakterien sind, die auf der Haut angetroffen werden.

**4.** Verwendung von Extrakten von Mikroorganismen nach Anspruch 3, **dadurch gekennzeichnet, dass** die Corynebakterien ausgewählt sind aus der Gruppe umfassend *Corynebacterium xerosis, Corynebacterium minutissum* und die lipophilen Corynebakterien der Gruppen CLC, JK und D2.

**5.** Verwendung von Extrakten von Mikroorganismen nach Anspruch 3, **dadurch gekennzeichnet, dass** die coryneformen Bakterien, die auf der Haut angetroffen werden, ausgewählt sind aus der Gruppe umfassend *Dermabacter hominis* und *Brevibacterium species*.

**6.** Kosmetische oder pharmazeutische Zusammensetzungen, umfassend Extrakte von Corynebakterien oder anderen coryneformen Bakterien, die auf der Haut angetroffen werden, wie in Anspruch 4 oder 5 definiert, **dadurch gekennzeichnet, dass** sie zur Hemmung von Körpergerüchen bestimmt sind.

7. Kosmetische oder pharmazeutische Zusammensetzungen nach Anspruch 6, **dadurch gekennzeichnet, dass** sie Oberflächen- und/oder exozelluläre Verbindungen von Corynebakterien umfassen.

8. Kosmetische oder pharmazeutische Zusammensetzungen nach einem der Ansprüche 6 und 7, **dadurch gekennzeichnet, dass** die Corynebakterien ausgewählt sind aus der Gruppe umfassend: *Corynebacterium xerosis, Corynebacterium minutissum* und die lipophilen Corynebakterien der Gruppen CLC, JK und D2.

9. Kosmetische oder pharmazeutische Zusammensetzungen nach Anspruch 6, **dadurch gekennzeichnet, dass** die coryneformen Bakterien, die auf der Haut angetroffen werden, ausgewählt sind aus der Gruppe umfassend *Dermabacter hominis* und *Brevibacterium species*.

10. Kosmetische oder pharmazeutische Zusammensetzungen nach einem der Ansprüche 6 bis 8, **dadurch gekennzeichnet, dass** sie Extrakte von Corynebakterien in einer Menge von 0,01 bis 20 Gewichts-%, bezogen auf die Gesamt-Zusammensetzung, umfassen.

11. Kosmetische oder pharmazeutische Zusammensetzungen nach Anspruch 10, **dadurch gekennzeichnet, dass** sie Extrakte von Corynebakterien in einer Menge von 0,5 bis 5 Gewichts-%, bezogen auf die GesamtZusammensetzung, umfassen.

**Claims**

1. Use of extracts of microorganisms, consisting of surface and/or exocellular compounds of said microorganisms, for preparing cosmetic or pharmaceutical compositions intended to inhibit body odours, namely underarm, perineal and foot odours, by saturating the sites of attachment of bacteria to the epidermal receptors.

2. Use of extracts of microorganisms according to Claim 1, **characterized in that** they are extracts of Gram[+] microorganisms.

3. Use of extracts of microorganisms according to Claim 1 or 2, **characterized in that** the microorganisms are corynebacteria or other coryneform bacteria found on the skin.

4. Use of extracts of microorganisms according to Claim 3, **characterized in that** the corynebacteria are chosen from the group comprising *Corynebackerium xerosis, Corynebacterium minutissum* and the lipophilic corynebacteria of the CLC, JK and D2 groups.

5. Use of extracts of microorganisms according to Claim 3, **characterized in that** the coryneform bacteria found on the skin are chosen from the group comprising *Dermabacter hominis* and *Brevibacterium species.*

6. Cosmetic or pharmaceutical compositions comprising extracts of corynebacteria or other coryneform bacteria found on the skin as defined in Claim 4 or 5, **characterized in that** they are intended to inhibit body odours.

7. Cosmetic or pharmaceutical compositions according to Claim 6, **characterized in that** they comprise surface and/ or exocellular compounds of corynebacteria.

8. Cosmetic or pharmaceutical compositions according to either of Claims 6 and 7, **characterized in that** the corynebacteria are chosen from the group comprising: *Corynebacterium xerosis, Corynebacterium minutissum* and the lipophilic corynebacteria of the CLC, JK and D2 groups.

9. Cosmetic or pharmaceutical compositions according to Claim 6, **characterized in that** the coryneform bacteria found on the skin are chosen from the group comprising *Dermabacter hominis* and *Brevibacterium species.*

10. Cosmetic or pharmaceutical compositions according to one of Claims 6 to 8, **characterized in that** they comprise extracts of corynebacteria in an amount of between 0.01% and 20% by weight relative to the total composition.

11. Cosmetic or pharmaceutical compositions according to Claim 10, **characterized in that** they comprise extracts of corynebacteria in an amount of between 0.5 and 5% by weight relative to the total composition.

EP 1 082 091 B1

## FIGURE 1

% adh. bact. aux kérat. HACAT

Légende: ■ Composés de surface / □ Composés exocellulaires

Valeurs: 50% : 46 / 34 — 25% : 63 / 49 — 12,5% : 66 / 53 — 6,2% : 60 / 50 — 3,1% : 70 / 70

% fract° microbienne

EP 1 082 091 B1

## FIGURE 2

% d'adhés. bact. aux kérat.
HACAT

composés de surface
composés exocellulaires

% fract° microb.

EP 1 082 091 B1

FIGURE 3

% adh.bact. aux kérat.
HACAT

composés de surface

composés exocellulaires

% fract° microb.

12,50%   6,25   3,1   1,5   0,7

64  55   73  61   76 72   86 78   87 87

11

EP 1 082 091 B1

## FIGURE 4

EP 1 082 091 B1

## FIGURE 5

% d'ah.bact. aux kèrat.
HACAT

81  88  92  91  98  64  55  73  61  75  72  74

100

80

40

20

0

12,50%   6,2 %   3,1 %   12,5 %   6,2 %   3,1 %

% fraction microb.

■ composés de surface
□ composés exocellulaires

2 h          4 h